# EUROPEAN PATENT SPECIFICATION

(11) **EP 3 324 971 B1**
(45) Date of publication and mention of the grant of the patent: **12.06.2024**
(21) Application number: 16827382.9
(22) Date of filing: 30.06.2016
(51) Int. Cl.: A61K 31/519, A61K 9/20, A61K 31/437, A61P 25/20

(54) **ZOLPIDEM COMPOSITION AND PROCESS FOR PREPARING THE SAME**
ZOLPIDEMZUSAMMENSETZUNG UND VERFAHREN ZUR HERSTELLUNG DAVON
COMPOSITION DE ZOLPIDEM ET SON PROCÉDÉ DE PRÉPARATION

(30) Priority: 23.07.2015 IN 2793MU2015
(43) Date of publication of application: 30.05.2018
(73) Proprietor: Athena Pharmaceutiques SAS, 78430 Louveciennes (FR)
(72) Inventor: CHAUDHARI, Mahendra B., Thane (west) 400602 (IN); WADHWANI, Jagdish S., Ulhasnagar 421003 (IN); CHAUDHARI, Amol Y., Jalgaon 425503 (IN); KATE, Sujata S., Thane (W) 400606 (IN)
(74) Representative: Grünecker Patent- und Rechtsanwälte PartG mbB
(86) International application number: PCT/IN2016/050211
(87) International publication number: WO 2017/013678

(56) References cited:
- US-A1- 2005 226 925
- US-A1- 2007 098 788
- US-A1- 2007 098 788
- US-A1- 2007 292 498
- US-A1- 2010 240 695
- US-A1- 2013 078 304
- US-A1- 2013 143 912
- US-A1- 2013 143 912

## Description

### FIELD OF THE INVENTION

The present invention relates to zolpidem compositions and process for preparing the same.

### BACKGROUND OF THE INVENTION

Zolpidem is a sedative that is used in the management of insomnia. It has been demonstrated to reduce sleep latency, increase sleep duration, and reduce night time awakenings. Zolpidem tablets and medications have been available for a long time; however the disintegration of conventional tablets is much slower resulting in decreased efficacy of the medicine. Further, due to the bitter taste of the conventional tablets, patients may have problems, particularly, elderly, patients with an impaired swallowing function, or patients with decreased saliva production. Accordingly, the oral intake of conventional tablets are a cumbersome process.

Also, orally disintegrating tablets (ODTs) are available. ODTs differ from conventional tablets in that they are designed to be dissolved/disintegrated in an oral cavity rather than swallowed whole. Accordingly, ODTs have been developed such that the same medicine can be orally administered in a simple and effective manner to children, adults and people with impaired swallowing function and similar conditions. Even a patient who is not suffering from any swallowing limitation will find it much easier to take an ODT as compared to a conventional tablet. An additional reason to use an orally disintegrating tablet is the convenience of a tablet that can be taken without water.

In view of ease of administration, the ODTs have a rapid disintegration rate as compared to conventional tablets. However, rapid disintegration properties and high tablet hardness are generally contradicting properties, and therefore ODTs cause chips and cracks of the tablets when divided because of insufficient tablet hardness and high friability. Due to high disintegration and low hardness, even the cracking or disintegration problems are faced in the course of production and/or distribution.

However, existing zolpidem ODT uses effervescent technology for taste masking. Further, also uses wet granulation method using ion exchange resins for taste masking. As used herein ion exchange resins are used for taste masking of various drugs by using wet granulation technique, wherein resins and drugs forms complex in presents of water/ solvent.

Also, preparation of the conventional taste masked zolpidem compositions requires several steps and requires use of one or more muco adhesive agents. Due to these problems the process for manufacturing a taste masked zolpidem compositions becomes time taking and complex.

Accordingly, what is required is a zolpidem compositions that has excellent disintegration and dissolution properties. Also, what is required is a taste masked zolpidem composition that can be easily prepared and administered. Further, what is required is a preparation process for zolpidem compositions that is simple, has uniformity reliability, and is time efficient.

US 2005/0226925 A1 discloses compositions for delivering hypnotic agents across the oral mucosa and methods of use thereof. US 2007/0098788 A1 discloses non-benzodiazepine hypnotic compositions. US 2013/0143912 A1 discloses sublingual zolpidem formulations.

### SUMMARY OF THE INVENTION

The subject matter of the invention is as set out in the appended claims.

One aspect of the invention relates to a zolpidem composition, comprising:
zolpidem tartrate and polacrillin potassium in a weight ratio of 1:8, wherein the zolpidem composition is obtainable by dry mixing of zolpidem tartrate and polacrillin potassium.

A further aspect of the invention relates to a process for preparation of a zolpidem composition, comprising:
dry mixing zolpidem tartrate and polacrillin potassium in a weight ratio of 1:8 to form a taste masked dry mix;
mixing the pharmaburst B2, lactose monohydrate, colloidal silicon dioxide anhydrous, a sweetener, and a flavorant to form a flavor blend;
blending of the taste masked dry mix and the flavor blend to form an intermediate blend;
mixing of intermediate blend with magnesium stearate to form ready to compress granules of the zolpidem composition.

Preferred embodiments are set forth in the subclaims.

In one aspect, the present invention provides zolpidem composition, comprising zolpidem tartrate and polacrillin potassium in a weight ratio of 1:8 (not according to the invention unless embraced by the claims). The zolpidem composition may further comprise pharmaburst B2, lactose monohydrate, colloidal silicon dioxide anhydrous, a sweetener, a flavorant, and magnesium stearate.

In another aspect, the present invention provides a process for preparation of a zolpidem composition. The process comprises: mixing zolpidem tartrate and polacrillin potassium in a weight ratio of 1:8 to form a taste masked dry mix; mixing the pharmaburst B2, lactose monohydrate, colloidal silicon dioxide anhydrous, a sweetener, and a flavorant to form a flavor blend; blending of the taste masked dry mix and the flavor blend to form an intermediate blend; mixing of intermediate blend with magnesium stearate to form ready to compress granules of the zolpidem composition; and compressing the ready to compress granules of the zolpidem composition to form a sublingual oral disintegrating tablet (not according to the invention unless embraced by the claims).

### BRIEF DESCRIPTION OF THE DRAWINGS

The advantages and features of the present invention will become better understood with reference to the following detailed description and claims taken in conjunction with the accompanying drawing, in which:
FIG. 1 illustrates a process flow for preparation of a zolpidem composition, in accordance with an exemplary embodiment of the present invention; and
FIG. 2 illustrates details of preparation of taste masked dry mix of process of FIG. 1;
FIG. 3 illustrates details of preparation of flavor blend of process of FIG. 1;
FIG. 4 illustrates details of preparation of intermediate blend of process of FIG. 1;
FIG. 5 illustrates details of preparation of granules of process of FIG. 1;
FIG. 6 illustrates details of compression of granules of process of FIG. 1 to form sublingual oral disintegrating tablet; and
FIG. 7 illustrates details of packing of sublingual oral disintegrating tablet.

### DETAILED DESCRIPTION OF THE INVENTION

The exemplary embodiments described herein detail for illustrative purposes are subject to many variations in structure and design. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items. Further, the terms, "a" and "an" herein do not denote a limitation of quantity, but rather denote the presence of at least one of the referenced item.

The present invention provides a zolpidem composition and a process for preparing the same. The zolpidem composition of the present invention has a taste masking achieved by only physical mixing of drug with an ion-exchange resin. As illustrated herein and described below, the preparation of the taste masked zolpidem composition requires fewer steps as compared to conventional taste masked zolpidem compositions. Also, the preparation process of the present invention employs direct compressible excipients without the use of any muco adhesive agents.

The zolpidem composition is further compressed using direct compression techniques and available as sublingual oral disintegrating tablet (ODT). Such compositions and formed ODTs are low dosage (5 milligrams, 10 milligrams) formulations. The sublingual ODT has excellent dissolution and disintegration properties and thus very quickly disperses in mouth without any need of water. Such ODTs are very handy for geriatric patients, patients with mental disabilities, and patients with impaired swallowing function.

The present invention provides a zolpidem composition comprising: zolpidem tartrate (drug) and polacrillin potassium (ion-exchange resin) in a weight ratio of 1:8.

The zolpidem composition further comprises Pharmaburst B2, lactose monohydrate, colloidal silicon dioxide anhydrous, a sweetener, a flavorant, and magnesium stearate.

As used herein, zolpidem tartrate is widely used to treat sleeping problems. Zolpidem tartrate is a white to off-white crystalline powder that is sparingly soluble in water, alcohol, and propylene glycol. As used in the present invention, zolpidem tartrate is obtained from Aarti Drugs Ltd.

Polacrilin potassium is the ion-exchange resin employed in the present invention. As used herein, the polacrilin potassium is an ion exchange resin used in oral pharmaceutical formulations as a tablet disintegrant. It is a weakly acidic cation exchange resin. Chemically, it is a partial potassium salt of a copolymer of methacrylic acid with divinyl benzene. As used in the present invention, polacrilin potassium is obtained from Corel Pharma Chem under the trade name Kyron T -134.

Pharmaburst B2 is employed in the present invention as a co-process excipient (diluent) for direct compression technique. As used herein, the pharmaburst B2 is used by the nutritional and pharmaceutical industry in the production of "quick dissolve" tablets. Pharmaburst B2 consists of 85 percent mannitol, 10 percent polyplasdone, 5 percent sorbitol (a sweetener), and less than one percent syloid. As used in the present invention, pharmaburst B2 is obtained from SPI Pharma.

Further, as used herein, lactose monohydrate as a second diluent is used. Lactose monohydrate is milk sugar (Chemically C₁₂H₂₂O₁₁). It is a disaccharide composed of one galactose and one glucose molecule. It is used to help form tablets because it has excellent compressibility properties. As used in the present invention, lactose monohydrate may be obtained from one of DMV, Fonterra Excipients and DFE Pharma. For example, lactose monohydrate is obtained from DFE Pharma under the Trade Name Supertab 30^{®} GR.

Colloidal silicon dioxide anhydrous is employed in the present invention as a glidant. As a glidant, colloidal silicon dioxide anhydrous serves as a material for improving powder flow since colloidal silicon dioxide is inert and doesn't dissolve in water. As used herein the colloidal silicon dioxide anhydrous refers to a fumed silica prepared by vapour-phase hydrolysis of a silicon compound, such as silicon tetrachloride. The product itself is usually a submicron, fluffy, light, loose, bluish-white, odourless and tasteless amorphous powder which is commercially available from a number of sources, including Cabot Corporation (under the trade name Cab-O-Sil^{®}); Degussa, Inc. (under the trade name Aerosil^{®}); Huber Engineered Materials (Huber GL100 and GL200^{®}); Wacker (Wacker HDK ^{®}); and E.I. DuPont & Co. As used in the present invention, colloidal silicon dioxide anhydrous is obtained from Evonik. Colloidal silicon dioxide is also commonly known as Aerosil, colloidal silica, fumed silica, light anhydrous silicic acid, silicic anhydride, and silicon dioxide fumed, among others. In one embodiment, the colloidal silicon dioxide used is Aerosil 200. A variety of commercial grades of colloidal silicon dioxide are produced by varying the manufacturing.

As used herein, the sweeteners can include aspartame, saccharin sodium, dipotassium glycyrrhizinate, stevia, thaumatin, acesulfame K, sucralose, and combinations of the foregoing. In one embodiment, the sweetener is aspartame. Aspartame is an artificial, non-saccharine sweetener used as a sugar substitute in some foods and beverages. Aspartame is a methyl ester of the aspartic acid/phenylalanine dipeptide. As used in the present invention, the aspartame is obtained from Nutra sweets.

The flavoring agents that can be used include natural and artificial flavors. These flavors may be one or more of synthetic flavor oils and flavoring aromatics, and/or oils, oleo resins and extracts derived from plants, leaves, flowers, fruits, etc., and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavorings can be used individually or in admixture. Commonly used flavors also include mints such as peppermint, artificial vanilla, cinnamon derivatives, and various fruit flavors, whether employed individually or in admixture. Flavorings such as aldehydes and esters including cinnamyl acetate, cinnamaldehyde, citral, diethylacetal, dihydrocarvyl acetate, eugenyl formate, p-methylanisole, and so forth may also be used. In one embodiment, the flavorant is Flavor- Black Current. As used in the present invention, the Flavor- Black Current is obtained from Firmenich. The Flavor- Black Current provides a good mouth feel.

Magnesium Stearate is employed in the present invention as a lubricant. Lubricants can be added to granules both during the final mixing phase before compression and during granulation. Among the traditional solid lubricants, calcium, magnesium, and zinc salts of stearic acid, partially hydrogenated vegetable oils, polyethylene glycols, polyoxyethylene monostearate, talc, sodium benzoate, sodium laurylsulfate, magnesium oxide can be used. In one embodiment lubricant is used in the blending applications for anti-adherent activity (i.e., prevent sticking to punch faces and die walls), glidant activity (i.e., improve the flowability of the powder or granules), and lubricant activity (i.e., reduce friction, transfer heat, and prevent corrosion during the process).

As used herein, magnesium stearate refers to a white powdered substance having the chemical formula Mg(C₁₈H₃₅O₂)₂. It is a salt containing two equivalents of stearate (the anion of stearic acid) and one magnesium cation (Mg²⁺). Magnesium stearate is used herein to serve as a lubricating medium for the composition. Magnesium stearate prevents ingredients from sticking to manufacturing equipment during the compression of chemical powders into solid tablets. Magnesium stearate has advantages over other lubricants because of its high melting temperature, high lubricity at a low concentration, large covering potential, general acceptance as safe, nontoxicity, and its excellent stability profile. As used in the present invention, the magnesium stearate is obtained from Ferro Corporation.

Further, the present invention provides a process for preparation of the zolpidem composition as described above (hereinafter referred to as process). The process employs a physical mixing, blending, lubricating, tablet compression and packing to obtain the zolpidem sublingual ODT.

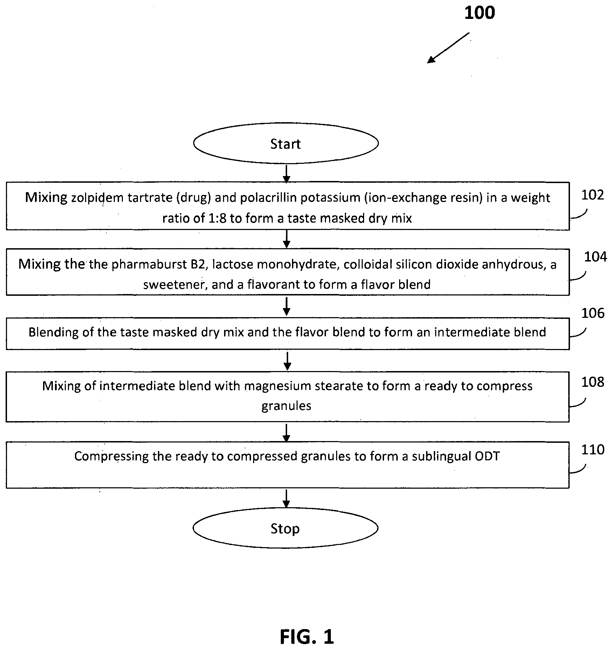

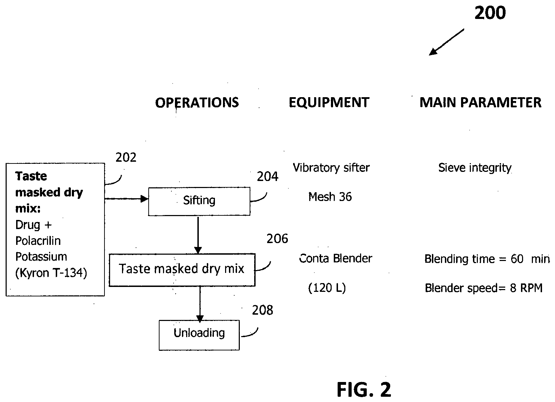

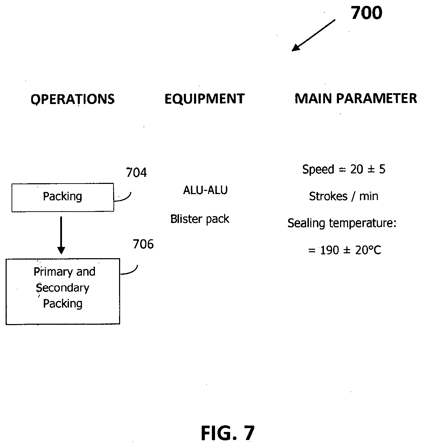

The preparation process 100 is described herein with reference to FIGS. 1-6; and packing of the zolpidem sublingual ODT is described herein with reference to FIG. 7. In FIG. 1, the primary process steps are illustrated, while in FIGS. 2, 3, 4, 5, and 6 illustrated are the details of steps in FIG. 1.

Referring to FIG. 1, at step 102, the process 100 initiates by mixing zolpidem tartrate (drug) and polacrillin potassium (ion-exchange resin) in a weight ratio of 1:8 to form a taste masked dry mix. At step 104, the process 100 comprises mixing the pharmaburst B2, lactose monohydrate, colloidal silicon dioxide anhydrous, a sweetener, and a flavorant to form a flavor blend. At step 106, the process 100 comprises blending of the taste masked dry mix and the flavor blend to form an intermediate blend. At step 108, the process 100 comprises mixing of intermediate blend with magnesium stearate to form a ready to compress granules of the zolpidem composition. At step 110, the ready to compress granules are compressed to form a sublingual ODT.

In one embodiment, at step 102, the zolpidem tartrate and polacrillin potassium are physically mixed for about 60 minutes using a rotating blender. Now, referring to FIG. 2, illustrated are the details of the dry mixing/physical mixing process of step 102 of FIG. 1 to form a taste masked dry mix through representations 202, 204, 206 and 208. At step 202, zolpidem tartrate is physically mixed with polacrillin potassium. At 204, the mix formed at 202 is sieved/sifted through a 36 mesh fitted with vibratory sifter to form a sifted mass of zolpidem tartrate and polacrillin potassium. Further, at 206, the sifted mass of zolpidem tartrate and polacrillin potassium are loaded and blended in the conta blender (rotating blender) to form the taste masked dry mix. In one embodiment, at 206 the blending is done in 120 litre conta blender for 60 minutes at the blender speed of 8 revolutions per minute (RPM). Thereafter, at 208, the taste masked dry mix is unloaded in a double poly bag lined HDPE container and labeled properly from inside and outside of container. Although a mesh screen of particular dimension is mentioned above, it will be evident to a person skilled in the art to use mesh sieves of varying dimensions for obvious variations. Also, although a particular volume of the blender and mixing time is mentioned, it will be evident to a person skilled in the art to use blender of varying dimensions and time for obvious variations.

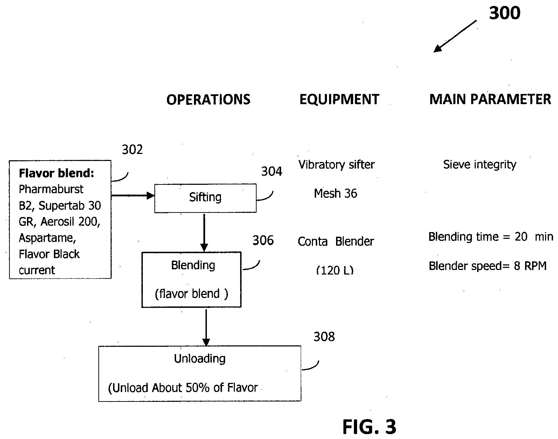

Now, referring to FIG. 3, illustrated are the details of the step 104 of FIG. 1 to form a flavor blend through representations 302, 304, 306 and 308. At step 302, Pharmaburst B2, lactose monohydrate, colloidal silicon dioxide anhydrous, the sweetener and the flavorant are mixed with each other. At 304, the mix formed at 302 is sieved/sifted through a 36 mesh fitted with vibratory sifter to form a sifted flavor blend. Further, at 306, the sifted flavor blend is loaded and blended in the conta blender to form flavor blend. In one embodiment, the blending is done in 120 litre conta blender for 20 minutes at the blender speed of 8 RPM. Thereafter, at 308, 50% of flavor blend is unloaded in a double poly bag lined HDPE container and labeled properly from inside and outside of container. Although a mesh screen of particular dimension is mentioned above, it will be evident to a person skilled in the art to use mesh sieves of varying dimensions for obvious variations. Also, although a particular volume of the blender and mixing time is mentioned, it will be evident to a person skilled in the art to use blender of varying dimensions and time for obvious variations.

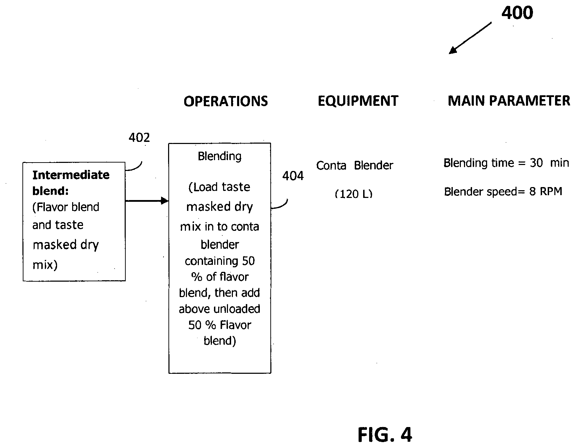

Now, referring to FIG. 4, illustrated are the details of the step 106 of FIG. 1 to form an intermediate blend through representations 402 and 404. Specifically, referring to 402 and 404 of FIG. 4, the intermediate blend is formed at 402 by blending of taste masked dry mix and flavor blend. More specifically, the first step comprises adding taste masked dry mix to 50% of the flavor blend in a contra blender bin. In a second step, the remaining 50% of the flavor blend is added. In one embodiment, at 404, the blending is done in 120 litre conta blender for 30 minutes at the blender speed of 8 RPM. Although a particular volume of the blender and mixing time is mentioned, it will be evident to a person skilled in the art to use blender of varying dimensions and time for obvious variations.

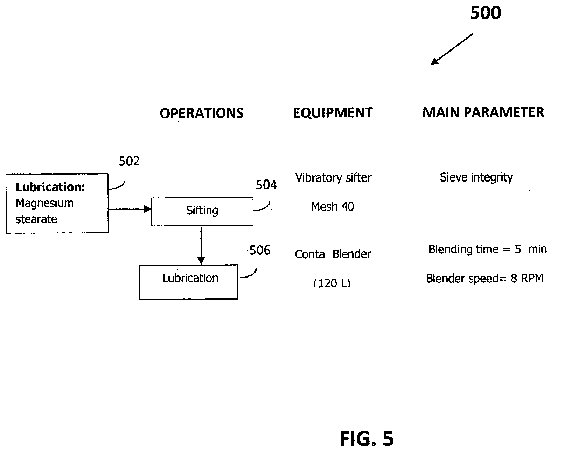

Now, referring to FIG. 5, illustrated are the details of the step 108 of FIG. 1 to form ready to compress granules of the zolpidem composition through representations 502, 504, and 506. At 502, magnesium stearate (as a lubricant) is added to the intermediate blend. At 504, the sieving/sifting is done through a 40 mesh fitted with vibratory sifter to form a sifted ready to compress granules. Further, the sifted ready to compress granules is blended with intermediate blend as illustrated at 506 in the conta blender to form ready to compress granules. At 506, the blending is done in 120 litre conta blender for 5 minutes at the blender speed of 8 RPM. Although a mesh screen of particular dimension is mentioned above, it will be evident to a person skilled in the art to use mesh sieves of varying dimensions for obvious variations. Also, although a particular volume of the blender and mixing time is mentioned, it will be evident to a person skilled in the art to use blender of varying dimensions and time for obvious variations.

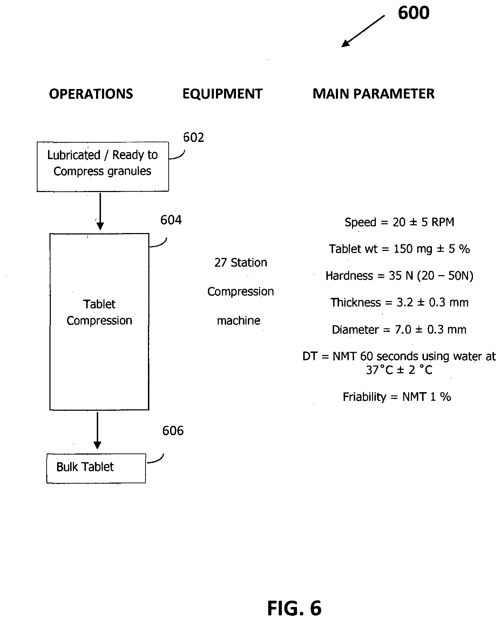

Now, referring to FIG. 6, illustrated are the details of the step 110 of FIG. 1 to compress the granules through representations 602, 604, and 606. At 602, 604, 606 the compression is performed using rotary tablet compression machine at a speed of about 25 revolutions per minute for form bulk tablet. Further, the compressing parameters for compressing the ready to compressed granules having different zolpidem compositions are described in the examples below.

The process may further comprise packing the zolpidem compositions. Referring to FIG. 7, at representations 704 and 706 the details of the packing are mentioned. The packaging will be performed in Aluminum - Aluminium blister pack machine with speed of about 20 + 5 strokes per minute and sealing heater temperature of about 190 + 20 degree centigrades.

The description of the zolpidem compositions of the present invention is further illustrated by the following non-limiting examples. However, a person skilled in the art would recognize that the examples described herein intended only to illustrate, not limit, the scope of the present invention.

### EXAMPLE 1

In Example 1, a process for preparing a 5 milligram (mg) zolpidem compositions is described. The process for preparing zolpidem compositions of the present invention was initiated by physically mixing 5mg of zolpidem tartrate with 40 mg of polacrillin potassium. The mix so formed is sieved/sifted through a 36 mesh fitted with vibratory sifter to form a sifted mass of zolpidem tartrate and polacrillin potassium. Further, the sifted mass of zolpidem tartrate and polacrillin potassium are loaded and blended in the conta blender (rotating blender) to form the taste masked dry mix. The blending is done in 120 litre conta blender for 60 minutes at the blender speed of 8 revolutions per minute (RPM). Thereafter, the taste masked dry mix is unloaded in a double poly bag lined HDPE container and labeled properly from inside and outside of container.

Next, the flavor blend is prepared by mixing 85.75 mg of Pharmaburst B2, 13 mg of lactose monohydrate, 1.5 mg of colloidal silicon dioxide anhydrous, 2.5 mg of sweetener and 0.75 mg of flavorant with each other. The mix so formed at is sieved/sifted through a 36 mesh fitted with vibratory sifter to form a sifted flavor blend. Further, the sifted flavor blend is loaded and blended in the conta blender to form flavor blend. In one embodiment, the blending is done in 120 litre conta blender for 20 minutes at the blender speed of 8 RPM. Thereafter, 50% of flavor blend is unloaded in a double poly bag lined HDPE container and labeled properly from inside and outside of container.

Next, the Intermediate blend is formed by blending of taste masked dry mix and flavor blend. More specifically, the first step comprises adding dry mix to 50% of the flavor blend in a contra blender bin. In a second step, the remaining 50% of the flavor blend is added. The blending is done in 120 litre conta blender for 30 minutes at the blender speed of 8 RPM.

Thereafter, ready to compress granules is prepared by adding 1.5 mg of magnesium stearate (as a lubricant) to the intermediate blend. The sieving/sifting is done through a 40 mesh fitted with vibratory sifter to form a sifted ready to compress granules. Further, the sifted ready to compress granules is blended with intermediate blend as illustrated in the conta blender to form ready to compress granules. The blending is done in 120 litre conta blender for 5 minutes at the blender speed of 8 RPM.

Finally, 5 mg of zolpidem composition is formed by compressing the ready to compress granules. The compression is performed using rotary tablet compression machine at a speed of about 25 revolutions per minute. Specifically, the ready to compress granules are compressed from 3.4 using rotatory tablet compression machine fitted with 7.0 mm round flat bevelled punches and dies using the tablet compression parameters described in the below Table 1. Also, referring to Table 1 below illustrated below are the parameters by which the lubricated pre- compression composition is compressed:-

**TABLE 1**

| Sr. No. | PARAMETERS | STANDARD | LIMITS |
|---|---|---|---|
| 1 | Appearance | White to off white, Round, flat, bevelled edged tablets plain on both the surfaces having characteristic odour. | |
| 2 | Punch description | 7 mm round flat bevelled edge punches plain on both the surfaces with corresponding dies | |
| 3 | Weight of 20 tablets | 3.0 g | 3.00 g ± 3% (2.91 - 3.09 g) |
| 4 | Individual tablet weight (n = 20) | 150 mg | 150 mg ±5% (143 - 158 mg) |
| 5 | Thickness (n = 5) | 3.2 mm | 3.2 mm ± 0.3 mm (2.9 - 3.5 mm) |
| 6 | Diameter (n = 5) | 7.0 mm | 7 mm ± 0.3 mm (6.7 - 7.3 mm) |
| 7 | Hardness (n = 5) | 35 N | 20 N - 50 N |
| 8 | Disintegration time | NMT 60 seconds using water at 370C ± 2°C without disc | NMT 60 seconds using water at 370C ± 2°C without disc |
| 9 | Friability | NMT 1.0 % | NMT 1.0% |

Although particular compression parameters are mentioned above, it will be evident to a person skilled in the art to modify parameters based on obvious variations.

Further, In Table 2 below, the different ingredients of the zolpidem composition are depicted along with details on pharmacopeial reference, the vendor from whom the ingredient was obtained, percent by weight of each ingredient and weight in mg of each ingredient. In tables below "(a)" denotes quantity of API shall be considered based on moisture content and % assay potency and "(b)" denotes quantity of API shall be adjusted against quantity of polacrilin potassium (kyron T -134)

**TABLE 2**

| Ingredients | Manufactur er | Quality Referenti al | % w/w | mg/ tab |
|---|---|---|---|---|
| Taste masked dry mix | | | | |
| Zolpidem Tartrate ^{(a)} (Particle size D90 ≤ 50µ ) | Aarti drugs Ltd | Ph.Eur | 3.33 | 5.0 |
| Polacrilin Potassium ^{(b)} (Kyron T -134) | Corel | USP | 26.66 | 40.0 |
| | Pharma | | | |
| | Chem | | | |

| Flavor Blend | | | | |
|---|---|---|---|---|
| Pharmaburst B2 | SPI Pharma | In-house | 57.17 | 85.75 |
| Lactose monohydrate (Supertab 30 GR) | DMV, Fonterra Excipients/ DFE Pharma | Ph.Eur | 8.67 | 13.0 |
| Aspartame | Nutrasweets | Ph.Eur | 1.67 | 2.5 |
| Colloidal silicon dioxide anhydrous (Aerosil 200) | Evonik | Ph.Eur | 1.0 | 1.5 |
| Flavor- Black Current (502009 AP0551) | Firmenich | In-house | 0.5 | 0.75 |

| Lubrication | | | | |
|---|---|---|---|---|
| Magnesium stearate | Ferro | Ph.Eur | 1.0 | 1.5 |
| | Corporation | | | |
| Total | | | 100.0 | 150.0 |

Specifically, the process and composition details described in Example 1 and Table 2 is used for preparing 5 mg zolpidem compositions. Multiple experiments were conducted to form 5 mg zolpidem compositions, i.e, to form zolpidem compositions comprising 5 mg of zolpidem Tartrate. For example, experiment for batches 1, 2 and 3 were conducted for forming 5 mg zolpidem compositions. It was found all batches resulted in substantially the same content indicating consistency in uniformity of content that is desirable for lower dosage compositions of 5 mg. Such properties for the different batches are illustrated in Tables below. Table 3 below illustrates the batch quantity for 2, 00,000 tablets in kilograms (kg). Table 4 below illustrates the analytical result of taste masked dry mix solution for different batches. Table 5 below illustrates the analytical result of unlubricated granules. In Tables 4 and 5, it can be concluded, blend uniformity test of Zolpidem Tartrate for all three batches shows that the drug content at all the ten points are within the specification and % RSD value less than 5, there by demonstrating adequate distribution of the drug. Table 6 illustrates the analytical result of lubricated granules. In Table 6, it can be concluded, assay and blend uniformity test for Zolpidem tartrate content was within the specification indicating uniform distribution of drug and also uniform distribution of particle size was observed in all three batches. Table 7 illustrates the analytical result of bulk tablets. In Table 7, it can be concluded, physical parameters and analytical results (Content of uniformity, Assay, dissolution, RS and Micro) of Zolpidem Tartrate were found within the specification for all three batches. Thus it concludes that direct compression process is suitable and reproducible for manufacturing of Zolpidem Tartrate sublingual orally disintegrating tablets. Table 8 illustrates the analytical result of pack tablets. In Table 8, it can be concluded, tablets were pack in Alu - Alu blister pack. Samples analysis results were complies with physical, chemical and Microbial limit tests specifications.

**TABLE 3**

| Ingredients | Batch Quantity for 2,00,000 tablets (kg) | | |
|---|---|---|---|
| | 1 | 2 | 3 |
| Zolpidem Tartrate ^{(a)} (Particle size D90 ≤ 50µ ) | 1.02^{(a)} | 1.03^{(a)} | 1.00^{(a)} |
| Polacrilin Potassium ^{(b)} (Kyron T -134) | 7.98^{(b)} | 7.97^{(b)} | 8.00^{(b)} |

| Flavor Blend | | | |
|---|---|---|---|
| Pharmaburst B2 | 17.15 | 17.15 | 17.15 |
| Lactose monohydrate (Supertab 30 GR) | 2.60 | 2.60 | 2.60 |
| Aspartame | 0.50 | 0.50 | 0.50 |
| Colloidal silicon dioxide anhydrous | 0.30 | 0.30 | 0.30 |
| (Aerosil 200) | | | |
| Flavor- Black Current (502009 AP0551) | 0.15 | 0.15 | 0.15 |

| Lubrication | | | |
|---|---|---|---|
| Magnesium stearate | 0.30 | 0.30 | 0.30 |
| Total | 30.00 | 30.00 | 30.00 |

**TABLE 4**

| Taste masked dry mix Yield | 1 | 2 | 3 |
|---|---|---|---|
| Theoretical yield | 8.98 kg | 8.98 kg | 8.98 kg |
| Practical yield in kg | 8.96 kg | 8.96 kg | 8.98 kg |
| % Practical yield | 99.77 % | 99.77 % | 100.0% |

**TABLE 5**

| Tests | 1 | 2 | 3 |
|---|---|---|---|
| Appearance | White to off white granules having characteristic odour | White to off white granules having characteristic odour | White to off white granules having characteristic odour |
| Blend uniformity: | Min: 96.0 % | Min: 97.4 % | Min: 97.8 % |
| 33.67 mg/g | | | |
| 30.30 to 37.04 mg/g (90.0 % to 110.0% of L.C) | | | |
| | Max: 100.8 % | Max: 109.0 % | Max: 101.6 % |
| | Avg: 98.8 % | Avg: 100.2% | Avg: 99.5 % |
| RSD: Not more than 5.0% | RSD: 1.5% | RSD: 3.2 % | RSD: 1.4% |

**TABLE 6**

| Tests | 1 | 2 | 3 |
|---|---|---|---|
| Appearance | White to off white granules having characteristic odour | White to off white granules having characteristic odour | White to off white granules having characteristic odour |
| Loss on drying: | 1.89 % | 2.19 % | 2.07 % |
| Not more than 5% | | | |
| Assay: | 33.81 mg/gm | 33.53 mg/gm | 33.15 mg/gm |
| 33.33 mg/g | | | |
| 31.66 - 35.0 mg/g (95 - 105% of L.C) | 101.4% | 100.6 % | 99.4 % |
| Particle size distribution: | 20 # = 0.12 % | 20 # = 0.12 % | 20 # = 0.10 % |
| | 35#= 0.40 % | 35#= 0.44 % | 35#= 0.28 % |
| | 40# = 0.60 % | 40# = 0.72 % | 40# = 0.84 % |
| | 50# = 1.85 % | 50 # = 2.03 % | 50 # = 2.64% |
| Not less than 95% particles passes through 500µ | 60 # = 4.00 % | 60 # = 4.24 % | 60 # = 5.02 % |
| | 80# = 11.16 % | 80# = 11.10 % | 80# = 25.25 % |
| | 100 # = 31.27 % | 100 # = 31.18 % | 100 # = 43.73 % |
| | 120 # = 38.71 % | 120 # = 38.57 % | 120 # = 56.17 % |
| | Below 120# = 99.22 % | Below 120# = 97.09 % | Below 120# = 99.96 % |
| Bulk density | 0.63 gm/ml | 0.61 gm/ml | 0.61 gm/ml |
| Tapped density | 0.85 gm/ml | 0.82 gm/ml | 0.83 gm/ml |
| Blend uniformity: | Min: 98.0 % | Min: 97.4 % | Min: 97.1 % |
| 33.33 mg/g | | | |
| 30.00 - 36.66 mg/g (90.0 % - 110.0 % of L.C) | Max: 101.8 % | Max: 101.2 % | Max: 101.9 % |
| | Avg: 100.8 % | Avg: 99.4 % | Avg: 99.0 % |
| RSD: Not more than 5.0% | RSD: 1.2% | RSD: 1.2% | RSD: 1.8% |
| Related substance: | ND | ND | ND |
| Zolpidem Impurity A - Not more than 0.20 % | | | |
| Each unknown impurities - Not more than 0.20% | BDL | BDL | BDL |
| Total impurities - Not more than 0.50% | BDL | BDL | BDL |
| Microbial Limit Test : | Less than 10 CFU/g | Less than 10 CFU/g | Less than 10 CFU/g |
| Total aerobic microbial unit: NMT 1000 cfu/g | | | |
| Total yeast and mold count: NMT 100 cfu/g | Less than 10 CFU/g | Less than 10 CFU/g | Less than 10 CFU/g |
| Following pathogens should be absent in 1 g: | | | |
| Escherichia coli | Absent in 1 g | Absent in 1 g | Absent in 1 g |
| staphylococcus aureus | Absent in 1 g | Absent in 1 g | Absent in 1 g |
| Pseudomonas aeruginosa | Absent in 1 g | Absent in 1 g | Absent in 1 g |
| salmonella species should be absent in 10 g. | Absent in 10 g | Absent in 10 g | Absent in 10 g |
| Theoretical weight of granules | | | |
| Quantity of granules | 29.42 kg | 29.44 kg | 29.36 kg |
| obtained | | | |
| Yield of granules | 99.80 % | 99.87 % | 99.73 % |
| Process Loss | 0.06 kg | 0.04 kg | 0.08 kg |

**TABLE 7**

| Tests | 1 | 2 | 3 |
|---|---|---|---|
| Description | White to off white, round, flat, bevelled edged tablets plain on both the surfaces having characteristic odour | White to off white, round, flat, bevelled edged tablets plain on both the surfaces having characteristic odour | White to off white, round, flat, bevelled edged tablets plain on both the surfaces having characteristic odour |
| Identification: | Complies | Complies | Complies |
| To comply with the test for Assay by HPLC | | | |
| The retention time of major peak in the chromatogram of the assay preparation corresponds to that in the chromatogram of the standard preparation, as obtained in assay. | | | |
| Average weight 150 mg ± 3% (145.5 - 154.5 mg) | 150.5 mg | 150.3 mg | 151.3 mg |
| Diameter: | 7.1 mm | 7.1 mm | 7.1 mm |
| 7.0 mm ± 0.3 mm (6.7 - 7.3 mm) | | | |
| Thickness: | 3.2 mm | 3.2 mm | 3.2 mm |
| 3.2 mm ± 0.3 mm (2.9 - 3.5 mm) | | | |
| Hardness: | 33N | 35 N | 38 N |
| Target 35 N (20 - 50 N) | | | |
| Friability: NMT 1.0% | 0.22 % | 0.22 % | 0.66 % |
| Loss on drying : | 1.93 % | 1.59 % | 2.36 % |
| NMT 5.0% | | | |
| Disintegration time: | 26 Sec | 27 sec | 26 sec |
| Not more than 60 seconds using water at 37°C±2°C without disc | | | |
| Uniformity of dosage unit: | Min: 99.4 % | Min: 97.4 % | Min: 96.2 % |
| The quantity of Zolpidem in each tablet should lie between 85.0% and 115.0% of label claim, Acceptance value <15.0 | Max: 105.8 % | Max: 101.2 % | Max: 100.4 % |
| | Avg: 102.2% | Avg: 98.8 % | Avg: 98.6% |
| | RSD: 1.9% | RSD: 1.3% | RSD: 1.3% |
| | AV: 5.3 | AV: 3.1 | AV: 3.0 |
| Dissolution: | Min: 95.0 % | Min: 95.2 % | Min: 94.6 % |
| (Paddle, 50 RPM, 900 ml 0.01N Hydrochloric acid) | Max: 101.4 % | Max: 100.0% | Max: 99.6% |
| | Avg: 98.3 % | Avg: 98.4 % | Avg: 97.7 % |
| | RSD: 2.3 | RSD: 1.6 | RSD: 1.9 |
| Assay: | 5.04 mg/tab | 4.98 mg/tab | 5.04 mg/tab |
| 5 mg/tablets (4.75 - 5.25 mg/tablets) 95.0 - 105.0 % | | | |
| | 100.9 % | 99.6 % | 100.8 % |
| Fineness of dispersion: | Complies | Complies | Complies |
| A smooth dispersion should produce which passes through a sieve screen with a nominal mesh aperture of 710 µm | | | |
| Uniformity of weight: | Complies | Complies | Complies |
| Not more than 2 of the individual masses deviate from the average mass by more than 5% and none deviates by more than 10% | | | |
| Related substance: | ND | ND | ND |
| Zolpidem Impurity A - Not more than 0.20 % | | | |
| Each unknown impurities - Not more than 0.20% | BDL | BDL | BDL |
| Total impurities - Not more than 0.50% | BDL | BDL | BDL |
| Microbial Limit Test : | Less than 10 CFU/g | Less than 10 CFU/g | Less than 10 CFU/g |
| Total aerobic microbial unit: NMT 1000 cfu/g | | | |
| Total yeast and mold count: NMT 100 cfu/g | Less than 10 CFU/g | Less than 10 CFU/g | Less than 10 CFU/g |
| Following pathogens should be absent in 1 g: | | | |
| Escherichia coli | Absent in 1 g | Absent in 1 g | Absent in 1 g |
| staphylococcus aureus | Absent in 1 g | Absent in 1 g | Absent in 1 g |
| Pseudomonas aeruginosa | Absent in 1 g | Absent in 1 g | Absent in 1 g |
| salmonella species should be absent in 10 g. | Absent in 10 g | Absent in 10 g | Absent in 10 g |
| Actual batch yield of compressed tablets | 98.65 % | 98.94 % | 98.51 % |

**TABLE 8**

| Tests | 1 | 2 | 3 |
|---|---|---|---|
| Description | White to off white, round, flat, bevelled edged tablets plain on both surface having characteristic odour, packed in Aluminum - Aluminum blister. | White to off white, round, flat, bevelled edged tablets plain on both surface having characteristic odour, packed in Aluminum - Aluminum blister. | White to off white, round, flat, bevelled edged tablets plain on both surface having characteristic odour, packed in Aluminum - Aluminum blister. |
| Identification: | Complies | Complies | Complies |
| To comply with the test for Assay by HPLC | | | |
| The retention time of major peak in the chromatogram of the assay preparation corresponds to that in the chromatogram of the standard preparation, as obtained in assay. | | | |
| Average weight 150 mg ± 3% (145.5 - 154.5 mg) | 150.67 mg | 152.02 mg | 151.46 mg |
| Diameter: | 7.1 mm | 7.1 mm | 7.1 mm |
| 7.0 mm ± 0.3 mm (6.7 - 7.3 mm) | | | |
| Thickness: | 3.2 mm | 3.2 mm | 3.2 mm |
| 3.2 mm ± 0.3 mm (2.9 - 3.5 mm) | | | |
| Hardness: | 41N | 42 N | 41N |
| Target 35 N (20 - 50 N) | | | |
| Friability: NMT 1.0% | 0.31 % | 0.39 % | 0.33 % |
| Fineness of dispersion: | Complies | Complies | Complies |
| A smooth dispersion should produce which passes through a sieve screen with a nominal mesh aperture of 710 µm. | | | |
| Loss on drying : | 1.83 % | 1.55 % | 1.90 % |
| NMT 5.0% | | | |
| Disintegration time: | 23 Sec | 18 sec | 23 sec |
| Not more than 60 seconds using water | | | |
| at 37°C±2°C without disc | | | |
| Uniformity of dosage unit: | Min: 99.4 % | Min: 97.4 % | Min: 96.2 % |
| The quantity of Zolpidem in each tablet should lie between 85.0% and 115.0% of label claim, Acceptance value <15.0 | | | |
| | Max: 105.8 % | Max: 101.2 % | Max: 100.4 % |
| | Avg: 102.2% | Avg: 98.8 % | Avg: 98.6% |
| | RSD: 1.9% | RSD: 1.3% | RSD: 1.3% |
| | AV: 5.3 | AV: 3.1 | AV: 3.0 |
| Leak Test: Complies as per current SOP No. PR/OP-29 | Complies | Complies | Complies |
| Dissolution: | Min: 99.4 % | Min: 98.2 % | Min: 99.8 % |
| (Paddle, 50 RPM, 900 ml 0.01N Hydrochloric acid) | Max: 101.6 % | Max: 101.4 % | Max: 102.4% |
| | Avg: 100.3 % | Avg: 99.6 % | Avg: 100.6 % |
| | RSD: 0.9 | RSD: 1.2 | RSD: 1.9 |
| Assay: | 4.97 mg/tab | 5.01 mg/tab | 4.98 mg/tab |
| 5 mg/tablets(4.75 - 5.25mg/tablets) 95.0-105.0 % | | | |
| | 99.3 % | 100.3 % | 99.6 % |
| Related substance: | ND | ND | ND |
| Zolpidem Impurity A - Not more than 0.20 % | | | |
| Each unknown impurities - Not more than 0.20% | BDL | BDL | BDL |
| Total impurities-Not more than 0.50% | BDL | BDL | BDL |
| Microbial Limit Test : | Less than 10 CFU/g | Less than 10 CFU/g | Less than 10 CFU/g |
| Total aerobic microbial unit: NMT 1000 cfu/g | | | |
| Total yeast and mold count: NMT 100 cfu/g | Less than 10 CFU/g | Less than 10 CFU/g | Less than 10 CFU/g |
| Following pathogens should be absent in 1 g: | | | |
| Escherichia coli | Absent in 1 g | Absent in 1 g | Absent in 1 g |
| staphylococcus aureus | Absent in 1 g | Absent in 1 g | Absent in 1 g |
| Pseudomonas aeruginosa | Absent in 1 g | Absent in 1 g | Absent in 1 g |
| salmonella species should be absent in 10 g. | Absent in 10 g | Absent in 10 g | Absent in 10 g |
| Packing % yield | 99.36 % | 99.0 % | 99.08 % |

### EXAMPLE 2

In Example 2, a process for preparing a 10 milligram (mg) zolpidem compositions is described. The process for preparing zolpidem compositions of the present invention was initiated by physically mixing 10 mg of zolpidem tartrate with 80 mg of polacrillin potassium. The mix so formed is sieved/sifted through a 36 mesh fitted with vibratory sifter to form a sifted mass of zolpidem tartrate and polacrillin potassium. Further, the sifted mass of zolpidem tartrate and polacrillin potassium are loaded and blended in the conta blender (rotating blender) to form the taste masked dry mix. The blending is done in 120 litre conta blender for 60 minutes at the blender speed of 8 revolutions per minute (RPM). Thereafter, the taste masked dry mix is unloaded in a double poly bag lined HDPE container and labeled properly from inside and outside of container

Next, the flavor blend is prepared by mixing 171.5 mg of Pharmaburst B2, 26 mg of lactose monohydrate, 3 mg of colloidal silicon dioxide anhydrous, 5 mg of sweetener and 1.5 mg of flavorant with each other. The mix so formed at is sieved/sifted through a 36 mesh fitted with vibratory sifter to form a sifted flavor blend. Further, the sifted flavor blend is loaded and blended in the conta blender to form flavor blend. In one embodiment, the blending is done in 120 litre conta blender for 20 minutes at the blender speed of 8 RPM. Thereafter, 50% of flavor blend is unloaded in a double poly bag lined HDPE container and labeled properly from inside and outside of container.

Next, the Intermediate blend is formed by blending of taste masked dry mix and flavor blend. More specifically, the first step comprises adding taste masked dry mix to 50% of the flavor blend in a contra blender bin. In a second step, the remaining 50% of the flavor blend is added. The blending is done in 120 litre conta blender for 30 minutes at the blender speed of 8 RPM.

Thereafter, ready to compress granules is prepared by adding 3 mg of magnesium stearate (as a lubricant) to the intermediate blend. The sieving/sifting is done through a 40 mesh fitted with vibratory sifter to form a sifted ready to compress granules. Further, the sifted ready to compress granules is blended with intermediate blend as illustrated in the conta blender to form ready to compress granules. The blending is done in 120 litre conta blender for 5 minutes at the blender speed of 8 RPM.

Finally, 10 mg of zolpidem compositions is formed by compressing the ready to compress granules. The compression is performed using rotary tablet compression machine at a speed of about 25 revolutions per minute. Specifically, the ready to compress granules are compressed from 3.4 using rotatory tablet compression machine fitted with 10.0 mm round flat bevelled punches and dies using the tablet compression parameters described in below table with tablet compression Machine speed of about 25 revolutions per minute. Also, referring to Table 9 below illustrated below are the parameters by which the lubricated pre- compression composition is compressed:-

**TABLE 9**

| Sr. No. | PARAMETERS | STANDARD | LIMITS |
|---|---|---|---|
| 1 | Appearance | White to off white, round, flat bevelled edged tablets plain on both the surface having characteristic odour. | |
| 2 | Punch description | 10 mm round flat beveled edge punches with corresponding dies | |
| 3 | Weight of 20 tablets | 6.0 g | 6.0 g ± 3% (5.82 g - 6.18 g) |
| 4 | Individual tablet weight | 300 mg | 300 mg ± 5 % (285 mg - 315 mg) |
| 5 | Thickness | 3.2 mm | 3.2 mm ± 0.3 mm |
| | | | (2.9 - 3.5 mm) |
| 6 | Diameter | 10.0 mm | 10.00 mm ± 0.3 mm (9.7 - 10.3 mm) |
| 7 | Hardness | 35 N | 20 N - 50 N |
| 8 | Disintegration time | NMT 60 second using water at 370C ± 2°C without disc | NMT 60 second using water at 370C ± 2°C without disc |
| 9 | Friability | NMT 1.0% | NMT 1.0 % |

Although particular compression parameters are mentioned above, it will be evident to a person skilled in the art to modify parameters based on obvious variations.

Further, In Table 10 below, the different ingredients of the zolpidem compositions are depicted along with details on pharmacopeial reference, the vendor from whom the ingredient was obtained, percent by weight of each ingredient and weight in mg of each ingredient. In tables below "(a)" denotes quantity of API shall be considered based on moisture content and % assay potency and "(b)" denotes quantity of API shall be adjusted against quantity of polacrilin potassium (kyron T - 134)

**TABLE 10**

| Ingredients | Manufactur er | Quality Referenti al | % w/w | mg/ tab |
|---|---|---|---|---|
| Taste masked dry mix | | | | |
| Zolpidem Tartrate ^{(a)} (Particle size D90 ≤ 50µ ) | Aarti drugs Ltd | Ph.Eur | 3.33 | 10.0 |
| Polacrilin Potassium ^{(b)} (Kyron T -134) | Corel Pharma Chem | USP | 26.66 | 80.0 |

| Flavor Blend | | | | |
|---|---|---|---|---|
| Pharmaburst B2 | SPI Pharma | In-house | 57.17 | 171.5 |
| Lactose monohydrate (Supertab 30 GR) | DMV, Fonterra Excipients/ DFE Pharma | Ph.Eur | 8.67 | 26.0 |
| Aspartame | Nutrasweets | Ph.Eur | 1.67 | 5.0 |
| Colloidal silicon dioxide anhydrous (Aerosil 200) | Evonik | Ph.Eur | 1.0 | 3.0 |
| Flavor- Black Current (502009 AP0551) | Firmenich | In-house | 0.5 | 1.5 |

| Lubrication | | | | |
|---|---|---|---|---|
| Magnesium stearate | Ferro Corporation | Ph.Eur | 1.0 | 3.0 |
| Total | | | 100.0 | 300.0 |

Specifically, the process and composition details described in Example 9 and Table 10 is used for preparing 10 mg zolpidem compositions. Multiple experiments were conducted to form 10 mg zolpidem compositions, i.e, to form zolpidem compositions comprising 10 mg of zolpidem Tartrate. For example, experiment for batches 4, 5 and 6 were conducted for forming 10 mg zolpidem compositions. It was found all batches resulted in substantially the same content indicating consistency in uniformity of content that is desirable for lower dosage compositions of 10 mg. Such properties for the different batches are illustrated in Tables below.

Table 11 below illustrates the batch quantity for 1,25,000 tablets in kilograms (kg). Table 12 below illustrates the analytical result of taste masked dry mix solution for different batches. Table 13 below illustrates the analytical result of unlubricated granules. In Tables 12 and 13, it can be concluded, blend uniformity test of zolpidem Tartrate for all three batches shows that the drug content at all the ten points are within the specification and % RSD value less than 5, there by demonstrating adequate distribution of the drug. Table 14 illustrates the analytical result of lubricated granules. In Table 14, it can be concluded, assay and blend uniformity test for zolpidem tartrate content was within the specification indicating uniform distribution of drug and also uniform distribution of particle size was observed in all three batches.

Table 15 illustrates the analytical result of bulk tablets. In Table 15, it can be concluded, physical parameters and analytical results (Content of uniformity, Assay, dissolution, RS and Micro) of Zolpidem Tartrate were found within the specification for all three batches. Thus it concludes that direct compression process is suitable and reproducible for manufacturing of Zolpidem Tartrate sublingual orally disintegrating tablets. Table 16 illustrates the analytical result of pack tablets. In Table 16, it can be concluded, tablets were pack in Alu - Alu blister pack. Samples analysis results were complies with physical, chemical and Microbial limit tests specifications.

**TABLE 11**

| Ingredients | Batch Quantity for 1,25,000 tablets (kg) | | |
|---|---|---|---|
| | 4 | 5 | 6 |
| Zolpidem Tartrate ^{(a)} (Particle size D90 ≤ 50µ ) | 1.274^{(a)} | 1.287^{(a)} | 1.250^{(a)} |
| Polacrilin Potassium ^{(b)} (Kyron T-134) | 9.976 ^{(b)} | 9.963^{(b)} | 10.000 ^{(b)} |

| Flavor Blend | | | |
|---|---|---|---|
| Pharmaburst B2 | 21.44 | 21.44 | 21.44 |
| Lactose monohydrate (Supertab 30 GR) | 3.25 | 3.25 | 3.25 |
| Aspartame | 0.625 | 0.625 | 0.625 |
| Colloidal silicon dioxide anhydrous (Aerosil 200) | 0.375 | 0.375 | 0.375 |
| Flavor- Black Current (502009 AP0551) | 0.188 | 0.188 | 0.188 |

| Lubrication | | | |
|---|---|---|---|
| Magnesium stearate | 0.375 | 0.375 | 0.375 |
| Total | 37.50 | 37.50 | 37.50 |

| TABLE 12 | | | |
|---|---|---|---|
| Taste masked dry mix Yield | 4 | 5 | 6 |
| Theoretical yield | 11.24 kg | 11.24 kg | 11.24 kg |
| Practical yield in kg | 11.22 kg | 11.24 kg | 11.22 kg |
| % Practical yield | 99.82 % | 100.0 % | 99.82% |

**TABLE 13**

| Tests | 4 | 5 | 6 |
|---|---|---|---|
| Appearance | White to off white granules having characteristic odour | White to off white granules having characteristic odour | White to off white granules having characteristic odour |
| Blend uniformity: 33.67 mg/g | | | |
| 30.30 to 37.04 mg/g (90.0 % to 110.0% of L.C) | Min: 97.8 % | Min: 97.0 % | Min: 95.0 % |
| | Max: 103.3 % | Max: 102.2 % | Max: 101.9 % |
| | Avg: 99.9 % | Avg: 99.1% | Avg: 97.9 % |
| RSD: Not more than 5.0% | RSD: 1.7% | RSD: 1.6 % | RSD: 2.1% |

**TABLE 14**

| Tests | 4 | 5 | 6 |
|---|---|---|---|
| Appearance | White to off white granules having characteristic odour | White to off white granules having characteristic odour | White to off white granules having characteristic odour |
| Loss on drying: | 2.35 % | 1.36% | 1.67 % |
| Not more than 5% | | | |
| Assay: | | | |
| 33.33 mg/g | 33.71 mg/gm | 33.10 mg/gm | 33.65 mg/gm |
| 31.66 - 35.0 mg/g (95 - 105% of L.C) | 101.2 % | 99.3 % | 101.0 % |
| | 20 # = 0.10% | 20 # = 0.12 % | 20 # = 0.08 % |
| | 35#= 0.38 % | 35#= 0.94 % | 35#= 0.78 % |
| Particle size distribution: | 40# = 0.88 % | 40# = 1.55 % | 40# = 1.34 % |
| | 50 # = 2.59 % | 50 # = 3.23 % | 50 # = 2.99 % |
| | 60 # = 5.32% | 60 # = 5.92% | 60 # = 5.61 % |
| Not less than 95% particles passes through 500µ | 80# = 25.58 % | 80# = 26.18 % | 80# = 25.85 % |
| | 100 # = 44.03 % | 100 # = 44.68 % | 100 # = 44.32 % |
| | 120 # = 56.41 % | 120 # = 57.13 % | 120 # = 56.76 % |
| | Below 120# = 99.36 % | Below 120# = 99.97 % | Below 120# = 99.64 % |
| Bulk density | 0.61 gm/ml | 0.60 gm/ml | 0.61 gm/ml |
| Tapped density | 0.81 gm/ml | 0.82 gm/ml | 0.84 gm/ml |
| Blend uniformity: | Min: 97.0 % | Min: 96.8 % | Min: 96.7 % |
| 33.33 mg/g | Max: 102.1 % | Max: 101.2 % | Max: 105.2 % |
| 30.00 - 36.66 mg/g | Avg: 99.9 % | Avg: 98.7 % | Avg: 99.5 % |
| (90.0 % - 110.0 % of L.C) | RSD: 1.7% | RSD: 1.6% | RSD: 2.4 % |
| RSD: Not more than 5.0% | | | |
| Related substance: | | | |
| Zolpidem Impurity A - Not more than 0.20 % | ND | ND | ND |
| Each unknown impurities - | BDL | BDL | BDL |
| Not more than 0.20% | | | |
| Total impurities-Not more than 0.50% | BDL | BDL | BDL |
| Microbial Limit Test : | Less than 10 CFU/g | Less than 10 CFU/g | Less than 10 CFU/g |
| Total aerobic microbial unit: NMT 1000 cfu/g | | | |
| Total yeast and mold count: NMT 100 cfu/g | Less than 10 CFU/g | Less than 10 CFU/g | Less than 10 CFU/g |
| Following pathogens should be absent in 1 g: | | | |
| Escherichia coli | Absent in 1 g | Absent in 1 g | Absent in 1 g |
| staphylococcus aureus | Absent in 1 g | Absent in 1 g | Absent in 1 g |
| Pseudomonas aeruginosa | Absent in 1 g | Absent in 1 g | Absent in 1 g |
| salmonella species should be absent in 10 g. | Absent in 10 g | Absent in 10 g | Absent in 10 g |
| Theoretical weight of granules | | | |
| Quantity of granules obtained | 36.70 kg | 36.80 kg | 36.84 kg |
| Yield of granules | 99.79 % | 99.86 % | 99.81 % |
| Process Loss | 0.08 kg | 0.05 kg | 0.07 kg |

**TABLE 15**

| Tests | 4 | 5 | 6 |
|---|---|---|---|
| Description | White to off white, round, flat, bevelled edged tablets plain on both the surfaces having characteristic odour | White to off white, round, flat, bevelled edged tablets plain on both the surfaces having characteristic odour | White to off white, round, flat, bevelled edged tablets plain on both the surfaces having characteristic odour |
| Identification: | Complies | Complies | Complies |
| To comply with the test for Assay by HPLC | | | |
| The retention time of major peak in the chromatogram of the assay preparation corresponds to that in the chromatogram of the standard preparation, as obtained in assay. | | | |
| Average weight 150 mg ± 3% (145.5 - 154.5 mg) | 301.6 mg | 295.6 mg | 298.2 mg |
| Diameter: | 10.1 mm | 10.1 mm | 10.1 mm |
| 7.0 mm ± 0.3 mm (6.7 - 7.3 mm) | | | |
| Thickness: | 3.2 mm | 3.2 mm | 3.2 mm |
| 3.2 mm ± 0.3 mm (2.9 - 3.5 mm) | | | |
| Hardness: | 41N | 42 N | 44 N |
| Target 35 N (20 - 50 N) | | | |
| Friability: NMT 1.0% | 0.44% | 0.31 % | 0.21 % |
| Loss on drying : NMT 5.0% | 2.22 % | 1.84 % | 2.65 % |
| Disintegration time: | 26 Sec | 26 sec | 28 sec |
| Not more than 60 seconds using water at 37°C±2°C without | | | |
| disc | | | |
| Uniformity of dosage unit: | | | |
| The quantity of Zolpidem in each tablet should lie between 85.0% and 115.0% of label claim, Acceptance value <15.0 | Min: 100.1 % | Min: 99.2 % | Min: 100.1 % |
| | Max: 103.8 % | Max: 104.2 % | Max: 104.1 % |
| | Avg: 101.5% | Avg: 99.2 % | Avg: 101.6% |
| | RSD: 1.4% | RSD: 1.8 % | RSD: 1.4% |
| | AV: 3.4 | AV: 4.5 | AV: 3.5 |
| Dissolution: | Min: 100.4 % | Min: 97.0 % | Min: 98.1 % |
| (Paddle, 50 RPM, 900 ml 0.01N Hydrochloric acid) | Max: 102.9 % | Max: 105.6 % | Max: 99.4% |
| | Avg: 101.9 % | Avg: 101.5 % | Avg: 98.6% |
| | RSD: 0.8 | RSD: 3.0 | RSD: 0.4 |
| Assay: | 10.05 mg/tab | 9.94 mg/tab | 9.99 mg/tab |
| 5 mg/tablets (4.75 - 5.25 mg/tablets) 95.0 - 105.0 % | | | |
| | 100.5 % | 99.4 % | 99.9 % |
| Fineness of dispersion: | Complies | Complies | Complies |
| A smooth dispersion should produce which passes through a sieve screen with a nominal mesh aperture of 710 µm | | | |
| Uniformity of weight: | Complies | Complies | Complies |
| Not more than 2 of the individual masses deviate from the average mass by more than 5% and none deviates by more than 10% | | | |
| Related substance: | ND | ND | ND |
| Zolpidem Impurity A - Not more than 0.20 % | | | |
| Each unknown | BDL | BDL | BDL |
| impurities - Not more than 0.20% | | | |
| Total impurities - Not more than 0.50% | BDL | BDL | BDL |
| Microbial Limit Test | Less than 10 CFU/g | Less than 10 CFU/g | Less than 10 CFU/g |
| Total aerobic microbial unit: NMT 1000 cfu/g | | | |
| Total yeast and mold count: NMT 100 cfu/g | Less than 10 CFU/g | Less than 10 CFU/g | Less than 10 CFU/g |
| Following pathogens should be absent in 1 g: | | | |
| Escherichia coli | Absent in 1 g | Absent in 1 g | Absent in 1 g |
| staphylococcus aureus | Absent in 1 g | Absent in 1 g | Absent in 1 g |
| Pseudomonas aeruginosa | Absent in 1 g | Absent in 1 g | Absent in 1 g |
| salmonella species should be absent in 10 g. | Absent in 10 g | Absent in 10 g | Absent in 10 g |
| Actual batch yield of compressed tablets | 98.48 % | 98.70 % | 98.65 % |

**TABLE 16**

| Tests | 4 | 5 | 6 |
|---|---|---|---|
| Description | White to off white, round, flat, bevelled edged tablets plain on both surface having characteristic odour, packed in Aluminum - Aluminum blister. | White to off white, round, flat, bevelled edged tablets plain on both surface having characteristic odour, packed in Aluminum - Aluminum blister. | White to off white, round, flat, bevelled edged tablets plain on both surface having characteristic odour, packed in Aluminum - Aluminum blister. |
| Identification: | Complies | Complies | Complies |
| To comply with the test for Assay by HPLC | | | |
| The retention time of major peak in the | | | |
| chromatogram of the assay preparation corresponds to that in the chromatogram of the standard preparation, as obtained in assay. | | | |
| Average weight 150 mg ± 3% (145.5 - 154.5 mg) | 301.61 mg | 301.13 mg | 300.52 mg |
| Diameter: | 10.1 mm | 10.1 mm | 10.1 mm |
| 7.0 mm ± 0.3 mm (6.7 - 7.3 mm) | | | |
| Thickness: | 3.2 mm | 3.2 mm | 3.2 mm |
| 3.2 mm ± 0.3 mm (2.9 - 3.5 mm) | | | |
| Hardness: | 41N | 40 N | 39 N |
| Target 35 N (20 - 50 N) | | | |
| Friability: NMT 1.0% | 0.26 % | 0.25 % | 0.35 % |
| Fineness of dispersion: | Complies | Complies | Complies |
| A smooth dispersion should produce which passes through a sieve screen with a nominal mesh aperture of 710 µm. | | | |
| Loss on drying : | 1.34% | 0.82 % | 1.09 % |
| NMT 5.0% | | | |
| Disintegration time: Not more than 60 seconds using water at 37°C±2°C without disc | 28 Sec | 27 sec | 30 sec |
| Uniformity of dosage unit: | Min: 100.1 % | Min: 99.2 % | Min: 100.1 % |
| | Max: 103.8 % | Max: 104.2 % | Max: 104.1 % |
| The quantity of Zolpidem in each | Avg: 101.5% | Avg: 99.2 % | Avg: 101.6% |
| | RSD: 1.4% | RSD: 1.8 % | RSD: 1.4% |
| tablet should lie between 85.0% and 115.0% of label claim, Acceptance value <15.0 | AV: 3.4 | AV: 4.5 | AV: 3.5 |
| Leak Test: | Complies | Complies | Complies |
| Complies as per current SOP No. PR/OP-29 | | | |
| Dissolution: | Min: 98.3 % | Min: 98.9 % | Min: 98.5 % |
| (Paddle, 50 RPM, 900 ml 0.01N Hydrochloric acid) | Max: 100.5 % | Max: 101.0 % | Max: 101.6 % |
| | Avg: 99.4 % | Avg: 99.8 % | Avg: 99.9 % |
| | RSD: 0.8 | RSD: 0.9 | RSD: 1.1 |
| Assay: | 9.96 mg/tab | 9.94 mg/tab | 9.90 mg/tab |
| 5 mg/tablets (4.75 - 5.25 mg/tablets) 95.0 - 105.0 % | | | |
| | 99.6 % | 99.4 % | 99.0 % |
| Related substance: | ND | ND | ND |
| Zolpidem Impurity A - Not more than 0.20 % | | | |
| Each unknown impurities - Not more than 0.20% | BDL | BDL | BDL |
| Total impurities-Not more than 0.50% | BDL | BDL | BDL |
| Microbial Limit Test | Less than 10 CFU/g | Less than 10 CFU/g | Less than 10 CFU/g |
| Total aerobic microbial unit: NMT 1000 cfu/g | | | |
| Total yeast and mold count: NMT 100 cfu/g | Less than 10 CFU/g | Less than 10 CFU/g | Less than 10 CFU/g |
| Following pathogens should be absent in 1 g: | | | |
| Escherichia coli | Absent in 1 g | Absent in 1 g | Absent in 1 g |
| staphylococcus | Absent in 1 g | Absent in 1 g | Absent in 1 g |
| aureus | | | |
| Pseudomonas aeruginosa | Absent in 1 g | Absent in 1 g | Absent in 1 g |
| salmonella species should be absent in 10 g. | Absent in 10 g | Absent in 10 g | Absent in 10 g |
| Packing % yield | 99.09 % | 99.08% | 99.92 % |

Further, multiple experiments were conducted to form 10 mg zolpidem compositions for having best taste masking effect with good mouth feel effect. For example, experiment for different batches were conducted for forming 10 mg zolpidem compositions. It was found that 1:8 ratio of zolpidem tartrate and polacrillin potassium is appropriate for taste masking with mixing time of 60 minutes. Further, it is also found that black current flavor is suitable for taste masking with good mouth feel effect. Such properties for the different batches are illustrated in Tables below. Table 17 illustrates the taste masking effect aspect based on ratio of drug to the ion-exchange resin. Table 18 below illustrates the taste masking effect with flavorant. Table 19 below illustrates the taste masking effect with mixing time.

**TABLE 17**

| Batch No. | Drug + Kyron T 134 ratio | Result |
|---|---|---|
| 7 | 1:8 | No bitter taste |
| 8 | 1:4 | Bitter taste |
| 9 | 1:2 | Very bitter taste |

**TABLE 18**

| Batch No. | Drug + Flavor | Result |
|---|---|---|
| 10 | No flavor | highly bitter |
| 11 | Strawberry flavor | slightly bitter |
| 12 | Black current flavor with 1% concentration | Bitter less with good mouth feel effect. |
| 13 | Black current flavor with 0.5% concentration | Bitter less with good mouth feel effect. |
| 14 | Black current flavor with | Bitter with no good mouth |
| | 0.25% concentration | feel effect. |

**TABLE 19**

| | Time for mixing | Result |
|---|---|---|
| Drug + Kyron T 134 ratio 1:8 | 60 min | No bitter taste |
| | 30 min | Bitter taste |
| | 15 min | Very bitter taste |

The foregoing descriptions of specific embodiments of the present invention have been presented for purposes of illustration and description. They are not intended to be exhaustive or to limit the present invention to the precise forms disclosed, and obviously many modifications and variations are possible in light of the above teaching. The embodiments were chosen and described in order to best explain the principles of the present invention and its practical application, to thereby enable others skilled in the art to best utilize the present invention and various embodiments with various modifications as are suited to the particular use contemplated. (It is understood that various omission and substitutions of equivalents are contemplated as circumstance may suggest or render expedient, but such are intended to cover the application or implementation without departing from the scope of the claims of the present invention).

## Claims

1. A zolpidem composition, comprising:
zolpidem tartrate and polacrillin potassium in a weight ratio of 1:8, wherein the zolpidem composition is obtainable by dry mixing of zolpidem tartrate and polacrillin potassium.

2. The zolpidem composition of claim 1, further comprising pharmaburst B2, lactose monohydrate, colloidal silicon dioxide anhydrous, a sweetener, a flavorant, and magnesium stearate.

3. The zolpidem composition of claim 2, wherein the sweetener is aspartame.

4. The zolpidem composition of claim 2, wherein the flavorant is Flavor-Black Current.

5. The zolpidem composition of claim 1, wherein the zolpidem tartrate and polacrillin potassium are dry mixed for about 60 minutes using a rotating blender.

6. The zolpidem composition of claim 1, wherein a sublingual oral disintegrating tablet of the zolpidem composition comprises 5 milligrams of zolpidem tartrate and 40 milligrams of polacrillin potassium.

7. The zolpidem composition of claim 1, wherein a sublingual oral disintegrating tablet of the zolpidem composition comprises 10 milligrams of zolpidem tartrate and 80 milligrams of polacrillin potassium.

8. A process for preparation of a zolpidem composition, comprising:
dry mixing zolpidem tartrate and polacrillin potassium in a weight ratio of 1:8 to form a taste masked dry mix;
mixing the pharmaburst B2, lactose monohydrate, colloidal silicon dioxide anhydrous, a sweetener, and a flavorant to form a flavor blend;
blending of the taste masked dry mix and the flavor blend to form an intermediate blend;
mixing of intermediate blend with magnesium stearate to form ready to compress granules of the zolpidem composition.

9. The process of claim 8, wherein the ready to compress granules of the zolpidem composition are compressed to form a sublingual oral disintegrating tablet.

10. The process of claim 9, wherein a sublingual oral disintegrating tablet of the zolpidem composition comprises 5 milligrams of zolpidem tartrate and 40 milligrams of polacrillin potassium.

11. The process of claim 9, wherein a sublingual oral disintegrating tablet of the zolpidem composition comprises 10 milligrams of zolpidem tartrate and 80 milligrams of polacrillin potassium.

## Patentansprüche

1. Zolpidem-Zusammensetzung, umfassend:
Zolpidemtartrat und Polacrillin-Kalium in einem Gewichtsverhältnis von 1:8, wobei die Zolpidem-Zusammensetzung durch Trockenmischen von Zolpidemtartrat und Polacrillin-Kalium erhältlich ist.

2. Die Zolpidem-Zusammensetzung nach Anspruch 1, die ferner Pharmaburst B2, Lactose-Monohydrat, kolloidales wasserfreies Siliciumdioxid, einen Süßstoff, einen Aromastoff und Magnesiumstearat umfasst.

3. Die Zolpidem-Zusammensetzung nach Anspruch 2, wobei der Süßstoff Aspartam ist.

4. Die Zolpidem-Zusammensetzung nach Anspruch 2, wobei der Aromastoff *Flavor-Black Current* ist.

5. Die Zolpidem-Zusammensetzung nach Anspruch 1, wobei das Zolpidemtartrat und das Polacrillin-Kalium etwa 60 Minuten lang unter Verwendung eines rotierenden Mischers trocken gemischt werden.

6. Die Zolpidem-Zusammensetzung nach Anspruch 1, wobei eine sublinguale orale Zerfallstablette der Zolpidem-Zusammensetzung 5 Milligramm Zolpidemtartrat und 40 Milligramm Polacrillin-Kalium umfasst.

7. Die Zolpidem-Zusammensetzung nach Anspruch 1, wobei eine sublinguale orale Zerfallstablette der Zolpidem-Zusammensetzung 10 Milligramm Zolpidemtartrat und 80 Milligramm Polacrillin-Kalium umfasst.

8. Verfahren zur Herstellung einer Zolpidem-Zusammensetzung, umfassend:
Trockenmischen von Zolpidemtartrat und Polacrillin-Kalium in einem Gewichtsverhältnis von 1:8 zur Bildung einer geschmacksmaskierten Trockenmischung;
Mischen von Pharmaburst B2, Laktose-Monohydrat, kolloidalem wasserfreien Siliziumdioxid, einem Süßstoff und einem Aromastoff zur Bildung einer Aromamischung;
Mischen der geschmacksmaskierten Trockenmischung und der Aromamischung, um eine Zwischenmischung zu bilden;
Mischen der Zwischenmischung mit Magnesiumstearat, um ein komprimierfähiges Granulat der Zolpidem-Zusammensetzung zu bilden.

9. Das Verfahren nach Anspruch 8, wobei das komprimierfähige Granulat der Zolpidem-Zusammensetzung komprimiert wird, um eine sublinguale orale Zerfallstablette zu bilden.

10. Das Verfahren nach Anspruch 9, wobei eine sublinguale orale Zerfallstablette der Zolpidem-Zusammensetzung 5 Milligramm Zolpidemtartrat und 40 Milligramm Polacrillin-Kalium umfasst.

11. Das Verfahren nach Anspruch 9, wobei eine sublinguale orale Zerfallstablette der Zolpidem-Zusammensetzung 10 Milligramm Zolpidemtartrat und 80 Milligramm Polacrillin-Kalium umfasst.

## Revendications

1. Composition de zolpidem, comprenant :
du tartrate de zolpidem et de la polacriline potassique sous un rapport en poids de 1:8, la composition de zolpidem pouvant être obtenue par mélange à sec de tartrate de zolpidem et de polacriline potassique.

2. Composition de zolpidem selon la revendication 1, comprenant en outre du pharmaburst B2, du lactose monohydraté, du dioxyde de silicium colloïdal anhydre, un édulcorant, un agent aromatisant, et du stéarate de magnésium.

3. Composition de zolpidem selon la revendication 2, l'édulcorant étant l'aspartame.

4. Composition de zolpidem selon la revendication 2, l'agent aromatisant étant l'arôme cassis.

5. Composition de zolpidem selon la revendication 1, le tartrate de zolpidem et la polacriline potassique étant mélangés à sec durant environ 60 minutes en utilisant un mélangeur rotatif.

6. Composition de zolpidem selon la revendication 1, un comprimé à désintégration orale et administration par voie sublinguale de la composition de zolpidem comprenant 5 milligrammes de tartrate de zolpidem et 40 milligrammes de polacriline potassique.

7. Composition de zolpidem selon la revendication 1, un comprimé à désintégration orale et administration par voie sublinguale de la composition de zolpidem comprenant 10 milligrammes de tartrate de zolpidem et 80 milligrammes de polacriline potassique.

8. Procédé de préparation d'une composition de zolpidem, comprenant les étapes consistant à :
mélanger à sec du tartrate de zolpidem et de la polacriline potassique sous un rapport en poids de 1:8 pour former un mélange à sec à goût masqué ;
mélanger le pharmaburst B2, le lactose monohydraté, le dioxyde de silicium colloïdal anhydre, un édulcorant, et un agent aromatisant pour former un mélange aromatisé ;
mélanger le mélange à sec à goût masqué et le mélange aromatisé pour former un mélange intermédiaire ;
mélanger le mélange intermédiaire avec du stéarate de magnésium pour former des granules prêts à compresser de la composition de zolpidem.

9. Procédé selon la revendication 8, les granules prêts à compresser de la composition de zolpidem étant compressés pour former un comprimé à désintégration orale et administration par voie sublinguale.

10. Procédé selon la revendication 9, un comprimé à désintégration orale et administration par voie sublinguale de la composition de zolpidem comprenant 5 milligrammes de tartrate de zolpidem et 40 milligrammes de polacriline potassique.

11. Procédé selon la revendication 9, un comprimé à désintégration orale et administration par voie sublinguale de la composition de zolpidem comprenant 10 milligrammes de tartrate de zolpidem et 80 milligrammes de polacriline potassique.
